(19)

**Europäisches Patentamt**
**European Patent Office**
**Office européen des brevets**

(11) **EP 4 102 416 B1**

(12) **EUROPEAN PATENT SPECIFICATION**

(45) Date of publication and mention
of the grant of the patent:
**21.05.2025 Bulletin 2025/21**

(21) Application number: **21305788.8**

(22) Date of filing: **09.06.2021**

(51) International Patent Classification (IPC):
**G06N 10/60** (2022.01)     **G16C 10/00** (2019.01)

(52) Cooperative Patent Classification (CPC):
**G06N 10/60; G16C 10/00**

(54) **METHOD FOR FINDING AN OPTIMAL QUANTUM STATE MINIMIZING THE ENERGY OF A HAMILTONIAN OPERATOR WITH A QUANTUM PROCESSOR BY USING A VQE METHOD, METHOD FOR DETERMINING A QUANTUM STATE OF A CHEMICAL COMPOUND AND METHOD FOR DETERMINING PHYSICAL QUANTUM PROPERTIES OF MATERIALS**

VERFAHREN ZUM FINDEN EINES OPTIMALEN QUANTENZUSTANDS UNTER MINIMIERUNG DER ENERGIE EINES HAMILTONIAN-OPERATORS MIT EINEM QUANTENPROZESSOR UNTER VERWENDUNG EINES VQE-VERFAHRENS, VERFAHREN ZUR BESTIMMUNG EINES QUANTENZUSTANDS EINER CHEMISCHEN VERBINDUNG UND VERFAHREN ZUR BESTIMMUNG PHYSIKALISCHER QUANTENEIGENSCHAFTEN VON MATERIALIEN

PROCÉDÉ POUR TROUVER UN ÉTAT QUANTIQUE OPTIMAL RÉDUISANT AU MINIMUM L'ÉNERGIE D'UN OPÉRATEUR HAMILTONIEN AVEC UN PROCESSEUR QUANTIQUE À L'AIDE D'UN PROCÉDÉ VQE, PROCÉDÉ DE DÉTERMINATION D'UN ÉTAT QUANTIQUE D'UN COMPOSÉ CHIMIQUE ET PROCÉDÉ PERMETTANT DE DÉTERMINER LES PROPRIÉTÉS QUANTIQUES PHYSIQUES DE MATÉRIAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.12.2022 Bulletin 2022/50**

(73) Proprietor: **Bull SAS**
**78340 Les Clayes-sous-Bois (FR)**

(72) Inventors:
• **AYRAL, Thomas**
**78000 VERSAILLES (FR)**
• **BESSERVE, Pauline**
**78000 VERSAILLES (FR)**

(74) Representative: **Plasseraud IP**
**104 Rue de Richelieu**
**CS92104**
**75080 Paris Cedex 02 (FR)**

(56) References cited:
• **NIKOLAY V TKACHENKO ET AL: "Correlation-Informed Permutation of Qubits for Reducing Ansatz Depth in VQE", ARXIV.ORG, CORNELL UNIVERSITY LIBRARY, 201 OLIN LIBRARY CORNELL UNIVERSITY ITHACA, NY 14853, 10 September 2020 (2020-09-10), XP081760136**
• **SAM MCARDLE ET AL: "Quantum computational chemistry", ARXIV.ORG, CORNELL UNIVERSITY LIBRARY, 201 OLIN LIBRARY CORNELL UNIVERSITY ITHACA, NY 14853, 30 August 2018 (2018-08-30), XP081585393**
• **PRAKASH VERMA ET AL: "Scaling Up Electronic Structure Calculations on Quantum Computers: The Frozen Natural Orbital Based Method of Increments", ARXIV.ORG, CORNELL UNIVERSITY LIBRARY, 201 OLIN LIBRARY CORNELL UNIVERSITY ITHACA, NY 14853, 21 April 2021 (2021-04-21), XP081922991**

## Description

### Technical Field

This disclosure pertains to the field of methods for finding an optimal quantum state minimizing the energy of a Hamiltonian operator with a quantum processor by using a Variational Quantum Eigensolver, VQE, method, which can be used for determining a quantum state of a chemical compound or for determining physical quantum properties of materials.

### Background Art

Currently, the performance of the quantum programs that can be executed on quantum processors is severely limited by their duration and complexity, in part due to the quantum noise affecting the quantum processors and the important depths of the quantum circuits implementing the quantum program. In particular, quantum programs used to compute the minimum of a cost function need to be tailored to respect the noise constraints to avoid a large accuracy loss. Existing methods require quantum circuit depths that are usually incompatible with the quantum processors noise constraints leading to poor accuracies in the determination of the minimum of a cost function.

Some methods have been developed in order to compute the minimum of a cost function, expressed as the expectation value of a Hamiltonian operator over a variational state, by using a variational method known as Variational Quantum Eigensolver (VQE) method. Generally speaking, this method is used to estimate the quantum ground state of a given Hamiltonian operator. The computing of the VQE method starts from a trial quantum state constructed using a quantum circuit defined by a set of parameters such as rotation gates' angles. The energy associated with the Hamiltonian operator is computed for this given instance of the trial circuit. Then, a classical minimizer proposes new values for the circuit's parameters. The process goes on with updated parameters being proposed by the classical minimizer based on the energy evaluations provided by the quantum computer until hopefully the minimal energy is returned. For this method to succeed, the trial quantum circuit must be expressive enough (typically, the more parametrized gates the quantum circuit encompasses, the more expressive it is), the minimization procedure must be feasible (the more parameters in the set of parameters of the quantum circuit, the harder the minimization) and the trial quantum state preparation and energy measurement must be accurate. However, on noisy quantum processors, the actual trial quantum state that is prepared departs from the expected perfect quantum state due to quantum noise, such that the measurement of its energy may also be altered, resulting in speed and accuracy problems in the convergence of the VQE methods.

Another method called PermVQE has been developed in the non-patent literature Correlation-Informed Permutation of Qubits for Reducing Ansatz Depth in VQE, Tkachenko et al arXiv preprint arXiv:2009.04996, 2020. In this paper, it is proposed to add an optimization loop to the VQE method that permutes qubits in order to solve for the qubit Hamiltonian that minimizes long-range correlations in the quantum ground state. The qubits are originally represented in an initial orbital basis, which is permuted in the optimization loop by the permutation of the qubits. The choice of permutations is based on mutual information, which is a measure of interaction between electrons in spin-orbitals. Encoding strongly interacting spin-orbitals into proximal qubits on a quantum processor with a nearest-neighbor connectivity (as is the case for e.g superconducting processors) naturally reduces the circuit depth needed to prepare the trial quantum state, as it does not require the insertion of additional SWAP gates (or other qubit routing methods) to satisfy the connectivity constraints.

It is also known from the numerous classical quantum chemistry and condensed-matter approaches how to exploit the freedom in selecting the basis in which the qubits are represented for improving the speed and/or accuracy of their algorithms. The prior art Quantum Theory of Many-Particle Systems. I. Physical Interpretations by Means of Density Matrices, Natural Spin-Orbitals, and Convergence Problems in the Method of Configurational Interaction, Physical Review volume 97 number 6, march 15, 1955, Per-Olov Lödwin, describes the concept of natural orbital basis to designate the basis in which the representation of a quantum state is the simplest, i.e. the basis that necessitates the least number of Slater determinants to represent the quantum state. In quantum mechanics, a Slater determinant is an expression that describes the wave function of a non-interacting multi-fermionic system. It satisfies anti-symmetry requirements, and consequently the Pauli principle, by changing sign upon exchange of two fermions. What is interesting from a quantum computing standpoint is that a Slater determinant is a state of the computational basis, i.e. a state that can be described with a single state on the qubits. As the number of Slater determinants in the representation of a quantum state dictates the complexity of the quantum circuit required to prepare it, minimizing it is crucial. However, the method is applicable only with classical processors, i.e. non-quantum processors. Moreover, the natural orbital basis one is interested in going to depends on the quantum ground state, which is not known since it is precisely what one is searching for. The natural orbital basis can thus be computed only a posteriori.

The prior art comprises the publication of Nikolay V Tkachenko et al: "Correlation-Informed Permutation of Qubits for Reducing Ansatz Depth in VQE", ARXIV.ORG, CORNELL UNIVERSITY LIBRARY, 201 OLIN LIBRARY CORNELL UNIVERSITY ITHACA, NY 14853, 10 September 2020 (2020-09-10).

## Summary

The invention at least partially remedies the shortcuts of the prior arts and optimizes the minimization of the energy associated with a Hamiltonian operator with a quantum processor.

According to a first aspect, it is proposed a method according to the appended claim 1.

Therefore, the VQE method is performed iteratively with an additional phase during which the orbital basis in which the Hamiltonian operator is provided gets updated based on the intermediate optimal quantum state determined by the VQE method. By iteratively determining an intermediate optimal quantum state and updating the orbital basis, the orbital basis progressively approaches the natural orbital basis, thereby progressively enhancing the expressivity of the quantum circuit used for preparing trial quantum states. The transformation of the minimization problem to the natural orbital basis endows the method with a guarantee that the minimization of the energy associated with the Hamiltonian operator is *in fine* performed in the most economical basis, enabling to compromise between the size of the quantum circuit used to prepare trial quantum states and its expressivity in the orbital basis.

The method enables for quantum circuits, preferably of low-depth, that may not be expressive enough in the initial orbital basis, to become more and more expressive as the method converges to the natural orbital basis. At the same time, since these predetermined quantum circuits may be of limited depth, they are robust to noise, thereby enhancing the accuracy of the minimization.

The following features, can be optionally implemented, separately or in combination one with the others:

the step of applying the VQE method is an iterative scheme in which the quantum processor is used in conjunction with a classical processor, the quantum processor preparing a trial quantum state for the Hamiltonian operator and performing measurements representative of the energy associated with the Hamiltonian operator for said trial quantum state, and the classical processor updating values of the parameters of the parametric quantum gates of the predetermined quantum circuit based on the measurements performed by the quantum processor, the iterative scheme being executed until a second predefined stopping criterion is satisfied, the VQE method returning the optimized values of the parameters;

the predetermined quantum circuit is a product quantum circuit comprising only one-qubit quantum gates in the form of rotations, or a quantum circuit comprising fSim quantum gates or a Low-Depth Circuit Ansatz, LDCA, quantum circuit;

a physical quantum state is encoded into a qubit state by means of the Jordan-Wigner transformation from which the Hamiltonian operator is decomposed accordingly in terms of qubit observables

the method is applied on a Hubbard model for which the Hamiltonian operator is provided;

the Hubbard model is a Hubbard model at half-filling;

the Hamiltonian operator is a second-quantized Hamiltonian;

the optimal quantum state corresponding to the eigenvector associated the lowest eigenvalue;

the molecule is a H2, LiH and/or H2O molecule;

the number of qubits in the predetermined quantum circuit corresponds to a number of spin-orbitals used to describe the molecule;

the predefined stopping criterion is a maximum number of iterations and/or a minimum change of a variance between the minimums of the energy associated with the Hamiltonian operator obtained after two consecutive iterations;

the parametric quantum gates comprise rotation quantum gates, and wherein the parameters associated with said rotation quantum gates comprise values of angles.

## Brief Description of Drawings

**Fig. 1**

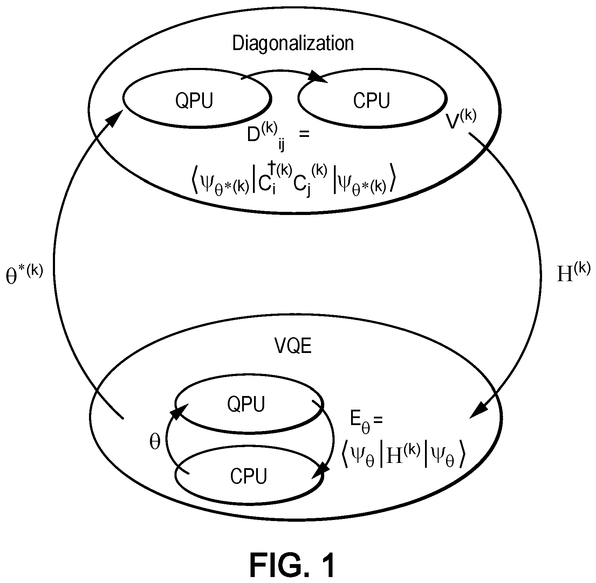

Fig. 1 is a flow diagram of the method for finding a minimum of a cost function with a noisy quantum processor.

**Fig. 2**

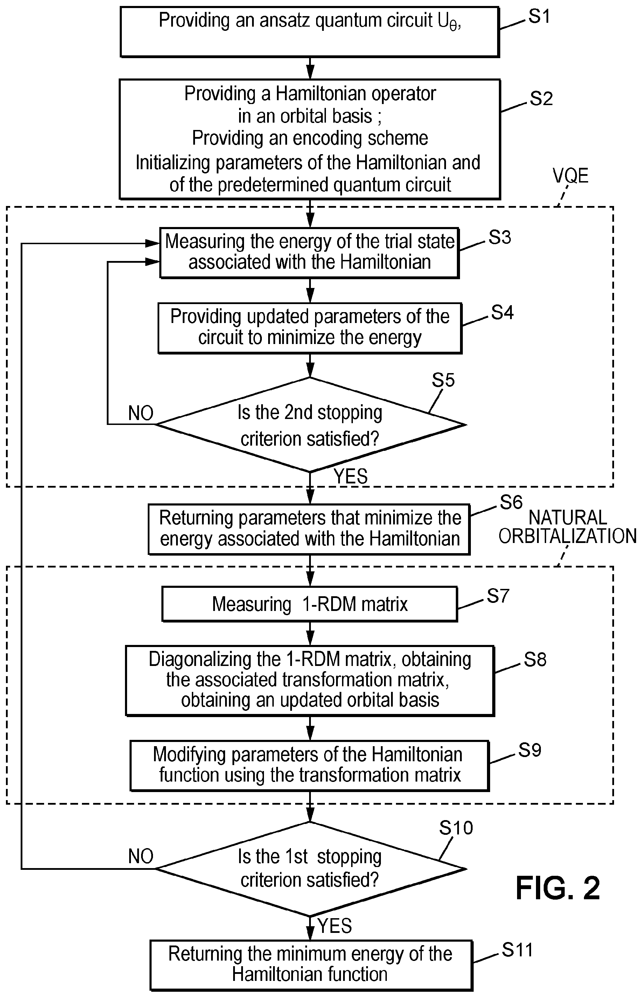

Fig. 2 is a flow chart illustrating some of the main steps of the method of figure 1.

**Fig. 3A to 3C**

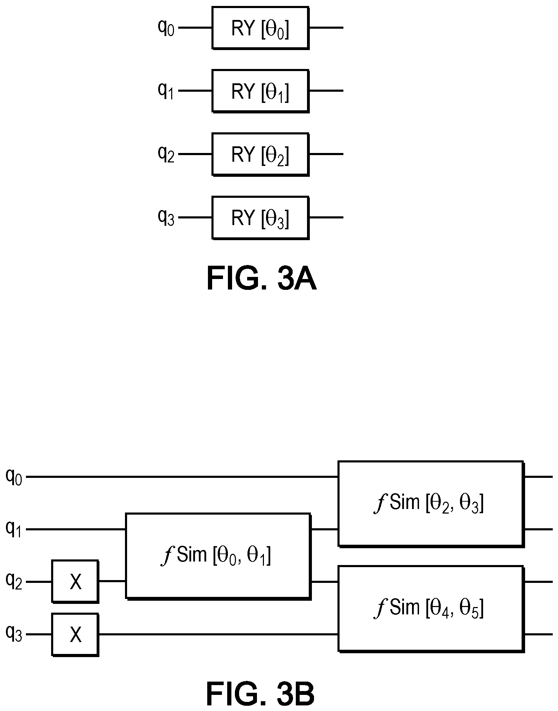

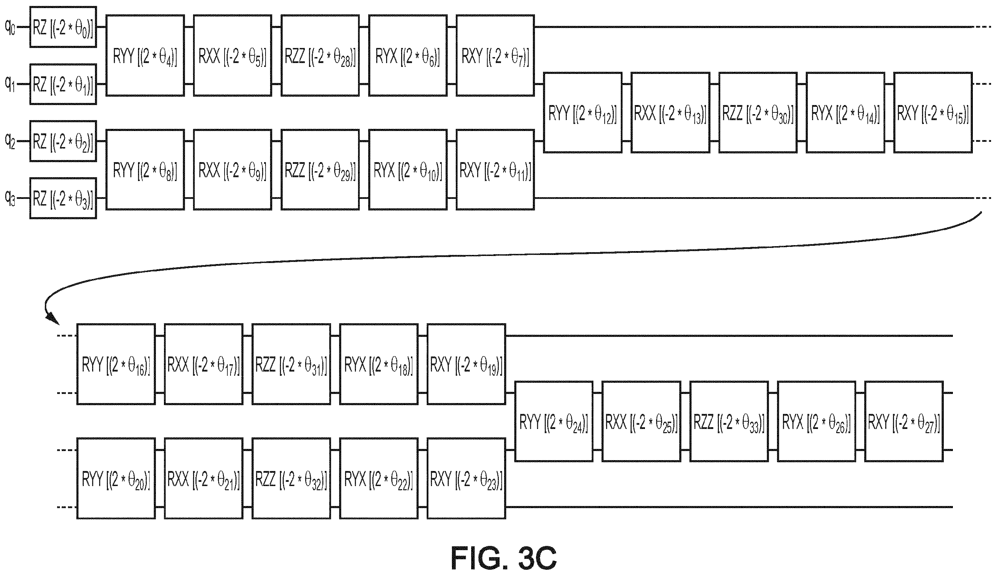

Fig. 3A illustrates an example of a quantum circuit comprising a product of rotations; fig. 3B illustrates an example of an fSim quantum circuit comprising fSim quantum gates and fig. 3C illustrates an example of a LDCA quantum circuit.

**Fig. 4A and 4B**

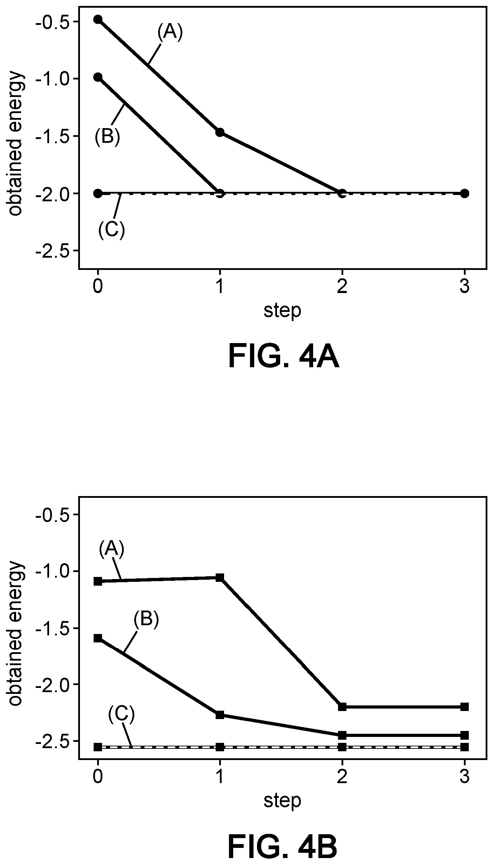

Figure 4A illustrates the performance of the method applied on the Hubbard model at half-filling on two sites, with $\mu =$

U/2 and U = 0; Figure 4B illustrates the performance of the method applied on the Hubbard model at half-filling on two sites, with $\mu$ = U/2 and U = 1.

### Fig. 5A and 5B

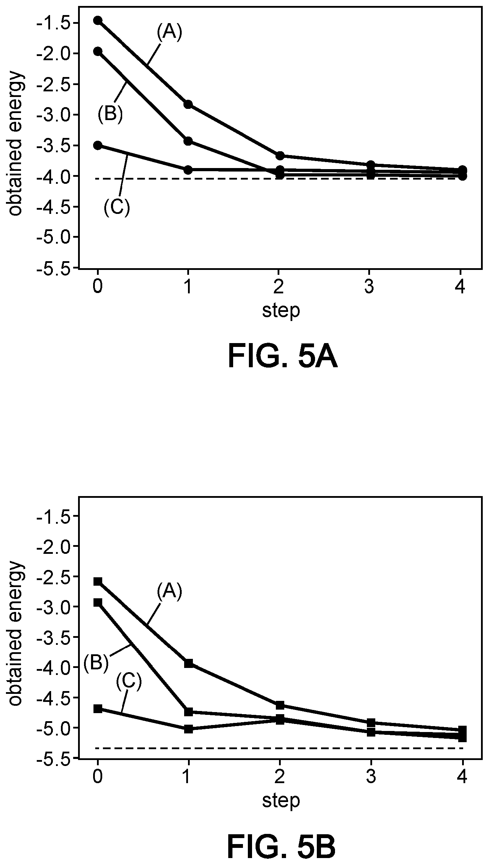

Figure 5A illustrates the performance of the method applied on the Hubbard model at half-filling on four sites, with $\mu$ = U/2 and U = 0, Figure 5B illustrates the performance of the method applied on the Hubbard model at half-filling on four sites, with $\mu$ = U/2 and U = 1.

### Fig. 6A and 6B

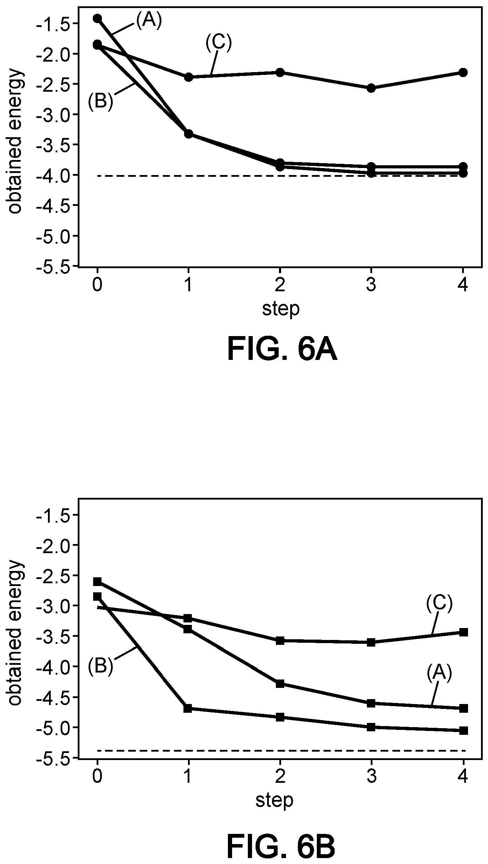

Figure 6A illustrates the performance of the method applied on the Hubbard model at half-filling on four sites, with $\mu$ = U/2 and U = 0, on a noisy processor with a depolarizing noise of magnitude 0.001 for the 1-qubit quantum gates and 0.01 for the 2-qubits quantum gates; figure 6B illustrates the performance of the method applied on the Hubbard model at half-filling on four sites, with $\mu$ = U/2 and U = 1, on a noisy processor with a depolarizing noise of magnitude 0.001 for the 1-qubit quantum gates and 0.01 for the 2-qubits quantum gates.

### Fig. 7A and 7B

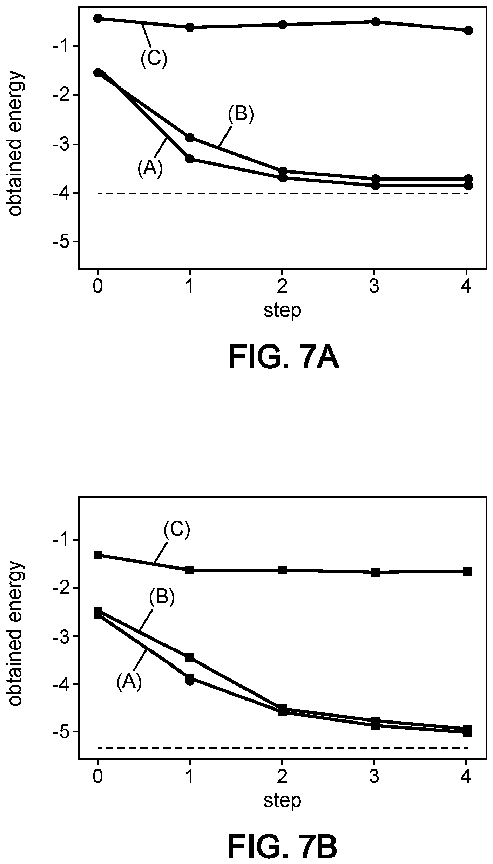

Figure 7A illustrates the performance of the method applied on the Hubbard model at half-filling on four sites, with $\mu$ = U/2 and U = 0, on a noisy processor a depolarizing noise of magnitude 0.005 for the 1-qubit quantum gates and 0.05 for the 2-qubits quantum gates; figure 7B illustrates the performance of the method applied on the Hubbard model at half-filling on four sites, with $\mu$ = U/2 and U = 1, on a noisy processor a depolarizing noise of magnitude 0.001 for the 1-qubit quantum gate and 0.01 for the 2-qubits quantum gates.

### Description of Embodiments

It is now referred to figures 1 and 2 respectively illustrating a flow diagram and a flow chart of the method for finding an optimal quantum state minimizing the energy of a Hamiltonian operator.

At step S1, a predetermined quantum circuit $U_\theta$, or simply "quantum circuit $U_\theta$" in the following specification, for providing trial quantum states $|\psi_\theta\rangle$ is provided. In the field of quantum mechanics, the quantum circuit $U_\theta$ can be referred to as an ansatz quantum circuit. The quantum circuit $U_\theta$ comprises at least parametric quantum gates associated with parameters $\theta$ to be optimized. The trial quantum states can be expressed as $|\psi_\theta\rangle = U_\theta |0\rangle^{\otimes M}$ where $|0\rangle^{\otimes M}$ is the initial quantum state of the M-qubit quantum processor.

In an embodiment, the parametric quantum gates of the quantum circuit $U_\theta$ comprise, for instance, quantum rotation gates, the parameters $\theta$ to be optimized comprising, for instance, angles of the quantum rotation gates.

In an embodiment, the method is computed on a Noisy Intermediate Scale Quantum (NISQ) processor, which is a quantum processing unit (QPU), comprising only a few tens of qubits and operating with relatively high error rates. Due to their limited capacity, NISQ processors are generally used in conjunction with classical processors (CPUs), for instance to minimize a cost function. The predetermined quantum circuit should comply with the constraints of the NISQ processor. In particular, the quantum circuit $U_\theta$ that is used to prepare the trial quantum state $|\psi_\theta\rangle = U_\theta|0\rangle^{\otimes M}$ (with M the number of qubits) should fulfill the connectivity and gateset constraints of the quantum processor, and more importantly be compatible with its decoherence properties. On the other hand, the quantum circuit $U_\theta$ needs to contain enough quantum gates so that the quantum state $|\psi_\theta\rangle$ it prepares can approximate with sufficient accuracy the expected quantum ground state, also called the optimal quantum state, to be found.

In an embodiment, the quantum circuit $U_\theta$ may be a "product" quantum circuit, comprising only one-qubit gates in the form of rotations as illustrated on figure 3A. In another embodiment, the quantum circuit $U_\theta$ may be an "fSim" quantum circuit, illustrated on figure 3B, and comprising fSim gates developed by the Google© company. In yet another embodiment, the quantum circuit $U_\theta$ may be the so-called "Low-Depth Circuit Ansatz", LDCA, circuit.

The product quantum circuit only produces product quantum states, also known as Slater determinants, and is adapted only to non-interacting Hamiltonian operators expressed in their diagonal basis.

The fSim quantum gates of the fSim quantum circuit conserve the number of excitations of a given quantum state.

The LDCA circuit was introduced in the following piece of literature Low-depth circuit ansatz for preparing correlated fermionic states on a quantum computer, Pierre-Luc Dallaire-Demers, Jonathan Romero, Libor Veis, Sukin Sim, Alán Aspuru-Guzik, January 4, 2018.

It should be noted that other types of quantum circuits may be used for preparing the trial quantum states, and the choice of specific type of quantum circuit corresponds to a specific embodiment of the present disclosure.

In an embodiment, the type of quantum gates, and the order of the qubits of the quantum circuit $U_\theta$ may be fixed, such that a qubit expressed in a scheme is always associated to the same qubit in the hardware, while the quantum circuit $U_\theta$ comprises at least parametric quantum gates which are tuned by the parameters $\theta$ to be optimized.

In another embodiment, the quantum circuit $U_\theta$ may comprise parametric quantum gates, as well as non-parametric quantum gates.

In yet another embodiment, only some of the parametric quantum gates may be tuned by the parameters $\theta$ to be optimized, while some other parametric quantum gates are associated with parameters which may not change during the implementation of the method.

At step S2, the Hamiltonian operator is provided in an orbital basis. In other words, the Hamiltonian operator is expressed in said orbital basis. The orbital basis in which the Hamiltonian operator is initially expressed is referred to as original orbital basis in the sequel.

In an embodiment, the Hamiltonian operator $H$ is a second-quantized Hamiltonian written as:

$$H = \sum_{pq} h_{pq}\, c_p^\dagger c_q + \frac{1}{2}\sum_{pqrs} h_{pqrs}\, c_p^\dagger c_q^\dagger c_r c_s$$

with $p,\,q,\,r,\,s \in M^4$, $M$ being the number of spin-orbitals.

In this embodiment, the Hamiltonian operator may represent the energy of an electronic system, such as a cloud of electrons in the field of an atomic potential in a molecule, with $c_p^\dagger$ and $c_q$ the creation and annihilation operators in the original orbital basis $\phi_p(r)$, $h_{pq}$ the overlap of atomic orbitals of a molecule and $h_{pqrs}$ the collision of two orbitals.

In this embodiment, the original orbital basis in which the Hamiltonian operator is expressed initially is the local orbital basis, i.e. the orbital basis that is centered on the atoms.

A generic cost function may be expressed as the expectation value over a quantum state of some quantum observable.

In this embodiment, the cost function to be minimized is the energy associated with the Hamiltonian operator, which is defined as:

$$E_\theta^{(k)} = \langle \psi(\theta)|\, H^{(k)}\, |\psi(\theta)\rangle$$

Mathematically, $E_\theta^{(k)}$ may then be expressed as

$$\sum_{pq} h_{pq}\, P_{pq} + \frac{1}{2}\sum_{pqrs} h_{pqrs} P_{pqrs}, \text{ with } P_{pq} = \langle\psi(\theta)|c_p^\dagger c_q|\psi(\theta)\rangle,$$

$$P_{pqrs} = \langle\psi(\theta)|c_p^\dagger c_q^\dagger c_r c_s|\psi(\theta)\rangle$$

and where $P_{pq}$ and $P_{pqrs}$ can be obtained from measurements with the quantum processor.

However, the Hamiltonian operator can be expressed differently, depending on the physical or chemical entity to be studied, without modifying the steps of the method for finding an optimal quantum state minimizing the energy associated with a Hamiltonian operator.

In particular, the Hamiltonian operator may be expressed with a form more general than the one used in this embodiment, which may be specific to chemistry and physics problems. A generic Hamiltonian would contain terms of higher order in the creation and annihilation operators $c_p^\dagger$ and $c_q$. Moreover, according to the system to be described with the cost function, the parameters $h_{pq}$ and $h_{pqrs}$ of the Hamiltonian may take different expressions.

Finally, step S2 may also comprise the initialization of the parameters $h_{pq}$ and $h_{pqrs}$ such that $h_{pq}^{(k=0)} = h_{pq}$ and $h_{pqrs}^{(k=0)} = h_{pqrs}$, with k being the current iteration, for the Hamiltonian operator $H^{(k=0)}$ expressed in the original orbital basis (with $c_p^{\dagger(k=0)} = c_p^\dagger$ and $c_q^{(k=0)} = c_q$).

The order of steps S1 and S2 is purely illustrative.

Steps S3 to S5 are then performed iteratively until a first predefined stopping criterion is met. The first predefined stopping criterion may be satisfied when a maximum number of iterations is performed and/or when the minimum energy associated with the Hamiltonian operator calculated during successive iterations no longer varies.

During steps S3 to S5, the VQE method is applied to find optimized values $\theta^{*(k)}$ of the parameters $\theta$ that yield an

intermediate optimal quantum state which minimizes the energy $E_\theta^{(k)} = \langle \psi(\theta) | H^{(k)} | \psi(\theta) \rangle$ associated with the Hamiltonian operator $H^{(k)}$ in the current orbital basis, with $|\psi(\theta)\rangle = U(\theta)|0\rangle$.

More specifically, the quantum processor (QPU on figure 1, step S3 on figure 2) prepares a trial state for the Hamiltonian operator and measures the values $P_{pq}$ and $P_{pqrs}$ representative of the energy associated with the Hamiltonian operator, as defined above. Then the quantum processor transmits these values to a classical processor (CPU on figure 1, step S4 on figure 2) that determines updated values for the parameters $\theta$ for preparing a trial quantum state that may further reduce the energy of the quantum state that is prepared, based on the measurements performed by the quantum processor.

As visible on figures 1 and 2, the VQE method is an iterative scheme which ends when a second predefined stopping criterion is satisfied.

In an embodiment, the second predefined stopping criterion may be satisfied when a maximum number of iterations is performed and/or when the optimized values $\theta^{*(k)}$ of the parameters $\theta$ updated during successive iterations no longer varies.

At step S6, the VQE method scheme returns the optimized values $\theta^{*(k)}$ of the parameters which yield and intermediate optimal quantum state $|\psi(\theta^{*(k)})\rangle$ minimizing the energy associated with the Hamiltonian operator in the current orbital basis, and transmits it to perform the diagonalization step illustrated on figure 1 and figure 2.

At step S7, a one particle reduced density matrix $D_{ij}^{(k)}$, also called 1-RDM, associated to the intermediate optimal quantum state $|\psi(\theta^{*(k)})\rangle$ is measured on the quantum processor (QPU on figure 1) such that

$$D_{ij}^{(k)}\big(\theta^{*(k)}\big) = \Big\langle \psi\big(\theta^{*(k)}\big) \Big| c_i^{\dagger(k)} c_j^{(k)} \Big| \psi\big(\theta^{*(k)}\big) \Big\rangle$$

, with $\theta^{*(k)}$ the optimized values of the parameters $\theta$ yielding the intermediate optimal quantum state $|\psi(\theta^{*(k)})\rangle$ minimizing the energy associated with the Hamiltonian operator expressed in the current orbital basis, and $c_i^{\dagger(k)}$ and $c_j^{(k)}$ the creation and annihilation operators in the current orbital basis (i.e. the 1-RDM matrix is measured in the current orbital basis), defined as:

$$c_i^{\dagger(k)} = \Sigma_p \left[ V_{pi}^{(k-1)} \right] c_p^{\dagger(k-1)}$$

$$c_j^{(k)} = \Sigma_p \left[ V_{pj}^{(k-1)} \right]^* c_p^{(k-1)}$$

At step S8, the classical processor (CPU on figure 1) diagonalizes the 1-RDM to obtain a transformation matrix $V^{(k)}$. An updated orbital basis is obtained which corresponds to the orbital basis in which the 1-RDM matrix is diagonal.

At step S9, the parameters $h_{pq}^{(k)}$ and $h_{pqrs}^{(k)}$ of the Hamiltonian operator are modified to $h_{pq}^{(k+1)}$ and $h_{pqrs}^{(k+1)}$ using the transformation matrix $V^{(k)}$ such that :

$$h_{pq}^{(k+1)} = \sum_{p'q'} V_{p'p}^{(k)} h_{p'q'}^{(k)} \left[ V^{(k)\dagger} \right]_{qq'}$$

$$h_{pqrs}^{(k+1)} = \sum_{p'q'r's'} V_{p'p}^{(k)} V_{q'q}^{(k)} h_{p'q'r's'}^{(k)} \left[ V^{(k)\dagger} \right]_{rr'} \left[ V^{(k)\dagger} \right]_{ss'}$$

The method is then iteratively processed to step S3 with the new parameters $h_{pq}^{(k+1)}$ and $h_{pqrs}^{(k+1)}$ which are used to compute the Hamiltonian operator $H^{(k+1)}$ expressed in the updated orbital basis (wherein the updated orbital basis at the end of iteration $k$ is referred to as current orbital basis at the beginning of iteration $k + 1$), used for the subsequent VQE method iteration, until the first predefined stopping criterion is satisfied (step S11).

When the first predefined stopping criterion (step S11) is satisfied, the minimum value of the energy associated with the Hamiltonian operator is considered to have been found and the optimal quantum state, which corresponds to one of the intermediate quantum states obtained, is returned.

In an embodiment, the optimal quantum state corresponds to the last intermediate optimal quantum state determined.

In another embodiment, the optimal quantum state is the intermediate quantum state which minimizes the most the energy associated with the Hamiltonian, among all the intermediate quantum states obtained during the implementation of the method.

Eventually, by performing the method iteratively, the updated orbital basis, in which the Hamiltonian operator is expressed, will come closer and closer to the natural orbital basis relative to the Hamiltonian's quantum ground state, allowing to reach this quantum ground state while limiting the number of gates in the quantum state preparation circuit. The main property of the natural orbital basis in quantum chemistry is that it is the orbital basis in which the description of a quantum state is the simplest, i.e. the basis that necessitates the least number of Slater determinants to represent the quantum state.

By representing the Hamiltonian operator in an orbital basis that is closer and closer to its natural orbital basis, at each iteration, it results in a higher and higher expressivity of the quantum circuit $U_\theta$. In other words, the transformation of the minimization problem to the natural orbital basis endows the method with a guarantee that the problem is placed in the most economical basis, due to the property that the natural orbital basis associated with the Hamiltonian's ground state is the basis in which it can be expressed in a minimal fashion.

As a consequence, the quantum circuit $U_\theta$ may not perform well in the initial orbital basis but it will perform better and better as the method iteratively converges to the natural orbital basis. Moreover, since the quantum circuit $U_\theta$ may be shallow, the minimization is robust to noise. Thus, the method allows using shallow quantum circuits, such as a product quantum circuit, or an fSim quantum circuit or a LDCA quantum circuit, as illustrated on figures 3A to 3C.

Mathematically, given a Hamiltonian $H$ and a quantum state $|\psi\rangle$, the natural orbital basis NO is defined as the basis that diagonalizes the one-particle reduced density matrix $D_{pq}$ 1-RDM

$$D_{pq} \equiv \langle \Psi | c_p^\dagger c_q | \Psi \rangle,$$

with $c_p^\dagger$ and $c_q$ the creation and annihilation operators in the original orbital basis $\phi_p(r)$.

More specifically, if $D_{pq} = V_{p\alpha} n_\alpha V_{\alpha q}^\dagger$, the natural orbitals are defined as

$$\tilde{c}_\alpha^\dagger \equiv \sum_p V_{p\alpha} c_p^\dagger.$$

The main property of the NO basis is that it is the basis where the quantum state $|\psi\rangle$ can be represented as a linear combination of the least number of Slater determinants, or, in quantum computing terms, of computational basis quantum states.

These quantum states are defined, in terms of creation operators, as

$$|\tilde{n}_1, \tilde{n}_2, ..., \tilde{n}_M\rangle \quad = \prod_{\alpha=1}^{M} \left(\tilde{c}_\alpha^\dagger\right)^{\tilde{n}_\alpha} |0\rangle^{\otimes M}$$

$$|n_1, n_2, ..., n_M\rangle \quad = \prod_{p=1}^{M} \left(c_p^\dagger\right)^{n_p} |0\rangle^{\otimes M}$$

The quantum state $|\psi\rangle$ can be represented either in the original basis, $\{|n_1, n_2, ... , n_M\rangle\}$:

$$|\Psi\rangle = \sum_{n_1, n_2, ..., n_M} a_{n_1, n_2, ..., n_M} |n_1, n_2, ..., n_M\rangle$$

Or in the natural orbital basis

$$|\Psi\rangle = \sum_{\tilde{n}_1, \tilde{n}_2, \ldots, \tilde{n}_M} \tilde{a}_{\tilde{n}_1, \tilde{n}_2, \ldots, \tilde{n}_M} |\tilde{n}_1, \tilde{n}_2, \ldots, \tilde{n}_M\rangle$$

The above-mentioned property means that the number of nonzero coefficients in this expansion is minimal for the natural orbital basis:

$$\#\{\tilde{a}_{\tilde{n}_1, \tilde{n}_2, \ldots, \tilde{n}_M}, |\tilde{a}_{\tilde{n}_1, \tilde{n}_2, \ldots, \tilde{n}_M}| > 0\}$$
$$\leq \#\{a_{n_1, n_2, \ldots, n_M}, |a_{n_1, n_2, \ldots, n_M}| > 0\}$$

As a consequence, the quantum circuit to prepare the quantum state $|\psi\rangle$ is simpler in the natural orbital basis than in the original orbital basis.

Figures 4A to 8B illustrate the performance of the method applied on the Hubbard model at half-filling, with $\mu = U/2$ on an increasing number of sites, the number of sites being 2, 3 and 4. The method has been applied on the three quantum circuits illustrated on figures 3A to 3C.

The Hubbard model is a model used in the field of condensed-matter physics to describe phase transitions in so-called correlated materials, for instance the transition between conducting and insulating systems, such as metals and Mott insulators.

In these specific and non-limiting examples, the method is used to study a chemical compound such as a molecule. The molecule may be a H2, LiH or H20 molecules.

The method further comprises providing an encoding scheme (step S2 of figure 2). More precisely, a mapping from physical states defined with fermionic variables to qubit states defined with spin variables is provided.

In an exemplary embodiment, this mapping is achieved through the Jordan-Wigner transformation.

Once the transformation is chosen, the Hamiltonian operator is decomposed accordingly in terms of qubit observables. The quantum circuit $U_\theta$ is also provided.

In an embodiment, the number of qubits in the quantum circuit $U_\theta$ is equal to the number of orbitals of the molecule.

In an embodiment, the number of quantum gates in the quantum circuit $U_\theta$ is also proportional to the number of qubits. For example, if a fSim quantum circuit is used, the number of gates scales as $O(Ml)$, with M the number of qubits in the circuit and l the number of fSim layers (l=1 in the non-limiting example provided). If a product quantum circuit is used, the number of quantum gates is M, M being the number of qubits in the circuit. If a LDCA quantum circuit is used, the number of gates scales as $O(Ml)$, with M the number of qubits in the circuit and l the number of layers of the LDCA routine employed (l=1 in the example provided).

Figures 4A and 4B show the simulated the performance of the method when computed on a noiseless quantum processor.

Figure 4A illustrates the performance of the method for each of the three quantum circuits, where the line (A) represents the performance for the product circuit, the line (B) represents the fSim circuit and the line (C) represents the LDCA circuit on a Hubbard model for two sites at half-filling where U = 0, and where the expected energy value, represented by the dotted line, is approximately equal to - 2.0 in this non limiting example.

Figure 4B illustrates the performance of the method for each of the three quantum circuits, where the line (A) represents the performance for the product circuit, the line (B) represents the fSim circuit and the line (C) represents the LDCA circuit on a Hubbard model for two sites at half-filling where U = 1, and where the expected energy value, represented by the dotted line, is approximately equal to - 2.5 in this non limiting example.

It can be seen from figures 4A and 4B that for U = 0 (figure 4A), the product circuit is able to recover the exact energy after a few steps. For U=1 (figure 4B), the product circuit does not reach the exact expected energy value because the expected optimal state is entangled due to interactions. However, the energy value reached by the product circuit gets lower as the orbital basis is rotated in a basis closer to the natural orbital basis.

The LDCA circuit reaches the expected energy value for U = 0 and U = 1 and at each step of the method. This is because the LDCA circuit is very expressive and is numerically able to reach the expected energy value for the size of this circuit.

The fSim circuit is intermediate with respect to the product circuit and LDCA circuit in terms of expressivity. After a few steps, the obtained energy value is closer to the expected energy value as the orbital basis is rotated closer to the natural orbital basis.

Figures 5A and 5B show the performance for the computing of the method on a Hubbard model at half-filling for 4 sites.

Figure 5A illustrates the performance of the method for each of the three quantum circuits, where the line (A) represents the performance for the product circuit, the line (B) represents the fSim circuit and the line (C) represents the LDCA circuit on a Hubbard model for four sites at half-filling where U = 0, and where the expected energy value, represented by the dotted line, is approximately equal to - 4.0 in this non limiting example.

Figure 5B illustrates the performance of the method for each of the three quantum circuits, where the line (A) represents the performance for the product circuit, the line (B) represents the fSim circuit and the line (C) represents the LDCA circuit on a Hubbard model for four sites at half-filling where U = 1, and where the expected energy value, represented by the dotted line, is approximately equal to - 4.5 in this non limiting example.

The illustrated performances show that the observations made with regards to figures 4A and 4B can be applied to figures 5A and 5B.

Figures 6A and 6B show simulated performance for the computing of the method on a noisy processor.

Since noise is going to penalize long circuits, going to the orbital basis that requires the shortest circuits will be advantageous.

Figure 6A illustrates the performance of the method for each of the three quantum circuits when computed on a noisy quantum processor, where the line (A) represents the performance for the product circuit, the line (B) represents the fSim circuit and the line (C) represents the LDCA circuit on a Hubbard model for four sites at half-filling where U = 0, where the depolarizing noise is equal to 0.001 for the 1-qubit quantum gates and 0.01 for the 2-qubits quantum gates and where the expected energy, represented by the dotted line, value is approximately equal to - 4.0 in this non limiting example.

Figure 6B illustrates the performance of the method for each of the three quantum circuits when computed on a noisy quantum processor, where the line (A) represents the performance for the product circuit, the line (B) represents the fSim circuit and the line (C) represents the LDCA circuit on a Hubbard model for four sites at half-filling where U = 1, where the depolarizing noise is of magnitude 0.001 for the 1-qubit quantum gates and 0.01 for the 2-qubits quantum gates and where the expected energy value, represented by the dotted line, is approximately equal to - 5.25 in this non limiting example.

It can be seen from figures 6A and 6B that the convergence of the obtained energy to the expected energy value is much faster for the quantum circuits with a small number of parameters such as the product and the fSim quantum circuits.

More precisely, both product and fSim quantum circuits retain their high accuracy at U= 0 (figure 6A), whereas at U = 1, noise slightly degrades their performances. However, due to the large depth of the LDCA circuit, the energy obtained by optimizing the latter in presence of noise is far off the expected energy value.

Figures 7A and 7B show the performance for the computing of the method on noisier quantum processors.

Figure 7A illustrates the performance of the method for each of the three quantum circuits when computed on a noisy quantum processor, where the line (A) represents the performance for the product circuit, the line (B) represents the fSim circuit and the line (C) represents the LDCA circuit on a Hubbard model for four sites at half-filling where U = 0, where the depolarizing noise is of magnitude0.005 for the 1-qubit quantum gates and 0.05 for the 2-qubits quantum gates and where the expected energy value, represented by the dotted line, is approximately equal to - 4.0 in this non limiting example.

Figure 7B illustrates the performance of the method for each of the three quantum circuits when computed on a noisy quantum processor, where the line (A) represents the performance for the product circuit, the line (B) represents the fSim circuit and the line (C) represents the LDCA circuit on a Hubbard model for four sites at half-filling where U = 1, where the depolarizing noise is of magnitude 0.005 for the 1-qubit quantum gates and 0.05 for the 2-qubits quantum gates and where the expected energy value, represented by the dotted line, is approximately equal to - 5.25 in this non limiting example.

The observations made with reference to figures 6A and 6B can be applied on the performance illustrated on figures 7A and 7B. More precisely, the accuracy of the obtained energy, compared to the expected energy value, for the product and fSim quantum circuits is maintained, while the energy reached by the LDCA circuit is far off the expected energy value, due to the large depth of the LDCA circuit.

In an embodiment (not shown), the method can be applied for a Hubbard model at half-filling for three sites.

## Claims

1. Method for finding an optimal quantum state minimizing the energy associated with a Hamiltonian operator with a quantum processor and a classical processor by using a Variational Quantum Eigensolver, VQE, method, wherein the Hamiltonian operator represents an energy of a molecule, the quantum processor comprising a predetermined quantum circuit for producing (S1) trial quantum states for the Hamiltonian operator, said predetermined quantum circuit comprising at least parametric quantum gates associated with one or more parameters to be optimized, the method comprising :

   providing (S2) the Hamiltonian operator in an orbital basis and iteratively, until a predefined stopping criterion is satisfied (S10):

- Performing (S3, S4, S5, S6) by the quantum processor in conjunction with the classical processor the VQE method to find optimized values for at least some of the parameters associated with the parametric quantum gates of the predetermined quantum circuit that yield an intermediate optimal quantum state which minimizes the energy associated with the Hamiltonian operator,
- measuring (S7) on the quantum processor a one particle reduced density matrix based on the intermediate optimal quantum state,
- diagonalizing (S8) by the classical processor the one particle reduced density matrix to obtain a transformation matrix, and determining an updated orbital basis in which the one particle reduced density matrix is diagonal, based on the transformation matrix,
- modifying (S9) the Hamiltonian operator by using the transformation matrix, to express the Hamiltonian operator in the updated orbital basis,

wherein, when the predefined stopping criterion is satisfied (S10), the method further comprises returning (S11), as the optimal quantum state minimizing the energy associated with the Hamiltonian operator, the intermediate optimal quantum state which minimizes the most the energy associated with the Hamiltonian operator.

2. Method according to claim 1, wherein the step of performing (S3, S4, S5, S6) the VQE method is an iterative scheme in which the quantum processor is used in conjunction with the classical processor, the quantum processor preparing (S3) a trial quantum state for the Hamiltonian operator and performing measurements representative of the energy associated with the Hamiltonian operator for said trial quantum state, and the classical processor updating (S4) values of the parameters of the parametric quantum gates of the predetermined quantum circuit based on the measurements performed by the quantum processor, the iterative scheme being executed until a second predefined stopping criterion is satisfied (S5), the VQE method returning (S6) the optimized values of the parameters.

3. Method according to claim 1 or 2, wherein the predetermined quantum circuit is a product quantum circuit comprising only one-qubit quantum gates in the form of rotations, or a quantum circuit comprising fSim quantum gates or a Low-Depth Circuit Ansatz, LDCA, quantum circuit.

4. Method according to any one of claims 1 to 3, wherein the method is applied on a Hubbard model for which the Hamiltonian operator is provided.

5. Method according to claim 4, wherein the Hamiltonian operator is a second-quantized Hamiltonian.

6. Method according to any of claims 4 to 5, wherein the optimal quantum state corresponding to the eigenvector associated the lowest eigenvalue.

7. Method according to claim 6, wherein the molecule is a $H_2$, $LiH$ and/or $H_2O$ molecule.

8. Method according to any of claims 6 and 7, wherein the number of qubits in the predetermined quantum circuit corresponds to a number of spin-orbitals used to describe the molecule.

9. Method according to any one of claims 1 to 8, wherein the predefined stopping criterion is a maximum number of iterations and/or a minimum change of a variance between the minimums of the energy associated with the Hamiltonian operator obtained after two consecutive iterations.

10. Method according to any of claims 1 to 9, wherein the parametric quantum gates comprise rotation quantum gates, and wherein the parameters associated with said rotation quantum gates comprise values of angles.

11. Method for determining a quantum state of a chemical compound, such as a molecule, comprising a method for finding an optimal quantum state minimizing the energy associated with a Hamiltonian operator with a quantum processor and a classical processor according to any one of claims 1 to 10, wherein an expectation value of the Hamiltonian operator over a given quantum state corresponds to the energy of said quantum state.

12. Method for determining physical properties of materials comprising a method for finding an optimal quantum state by minimizing the energy associated with a Hamiltonian operator with a quantum processor and a classical processor according to any of claims 1 to 10.

**Patentansprüche**

1. Verfahren zum Auffinden eines optimalen Quantenzustands, welcher die mit einem Hamilton-Operator assoziierte Energie minimiert, mit einem Quantenprozessor und einem klassischen Prozessor, unter Verwendung eines Variation-Quantum-Eigensolver-, VQE,-Verfahrens, wobei der Hamilton-Operator eine Energie eines Moleküls repräsentiert, wobei der Quantenprozessor eine vorbestimmte Quantenschaltung zum Erzeugen (S1) von Test-Quantenzuständen für den Hamilton-Operator umfasst, wobei die vorbestimmte Quantenschaltung wenigstens parametrische Quantengatter umfasst, welche mit einem oder mehreren zu optimierenden Parametern assoziiert sind, wobei das Verfahren umfasst:

   Bereitstellen (S2) des Hamilton-Operators in einer Orbitalbasis und iterativ, bis ein vordefiniertes Stoppkriterium erfüllt wird (S10):

   - Durchführen (S3, S4, S5, S6), durch den Quantenprozessor in Verbindung mit dem klassischen Prozessor, des VQE-Verfahrens, um optimierte Werte für wenigstens einige der Parameter zu finden, welche mit den parametrischen Quantengattern der vorbestimmten Quantenschaltung assoziiert sind und welche einen optimalen Zwischen-Quantenzustand ergeben, welcher die mit dem Hamilton-Operator assoziierte Energie minimiert,
   - Messen (S7), an dem Quantenprozessor, einer reduzierten Dichtematrix eines Partikels auf Grundlage des optimalen Zwischen-Quantenzustands,
   - Diagonalisieren (S8), durch den klassischen Prozessor, der reduzierten Dichtematrix eines Partikels, um eine Transformationsmatrix zu erhalten, und Bestimmen einer aktualisierten Orbitalbasis, in welcher die reduzierte Dichtematrix eines Partikels diagonal ist, auf Grundlage der Transformationsmatrix,
   - Modifizieren (S9) des Hamilton-Operators unter Verwendung der Transformationsmatrix, um den Hamilton-Operator in der aktualisierten Orbitalbasis auszudrücken,

   wobei, wenn das vordefinierte Stoppkriterium erfüllt ist (S10), das Verfahren ferner ein Zurückführen (S11), als den optimalen Quantenzustand, welcher die mit dem Hamilton-Operator assoziierte Energie reduziert, des optimalen Zwischen-Quantenzustands umfasst, welcher die mit dem Hamilton-Operator assoziierte Energie am meisten minimiert.

2. Verfahren nach Anspruch 1, wobei der Schritt eines Durchführens (S3, S4, S5, S6) des VQE-Verfahrens ein iteratives Schema ist, in welchem der Quantenprozessor in Verbindung mit dem klassischen Prozessor verwendet wird, wobei der Quantenprozessor (S3) einen Test-Quantenzustand für den Hamilton-Operator vorbereitet und für den Test-Quantenzustand Messungen durchführt, welche für die mit dem Hamilton-Operator assoziierte Energie repräsentativ sind, und wobei der klassische Prozessor Werte der Parameter der parametrischen Quantengatter der vorbestimmten Quantenschaltung auf Grundlage der durch den Quantenprozessor durchgeführten Messungen aktualisiert (S4), wobei das iterative Schema ausgeführt wird bis ein zweites vordefiniertes Stoppkriterium erfüllt ist (S5), wobei das VQE-Verfahren die optimierten Werte der Parameter zurückführt (S6).

3. Verfahren nach Anspruch 1 oder 2, wobei die vorbestimmte Quantenschaltung eine Produkt-Quantenschaltung, welche lediglich Ein-Qubit-Quantengatter in der Form von Rotationen umfasst, oder eine Quantenschaltung, welche fSim-Quantengatter umfasst, oder eine Low-Depth-Circuit-Ansatz-,LDCA,-Quantenschaltung ist.

4. Verfahren nach einem der Ansprüche 1 bis 3, wobei das Verfahren auf ein Hubbard-Modell angewendet wird, für welches der Hamilton-Operator bereitgestellt ist.

5. Verfahren nach Anspruch 4, wobei der Hamilton-Operator ein Hamilton einer zweiten Quantisierung ist.

6. Verfahren nach einem der Ansprüche 4 bis 5, wobei der optimale Quantenzustand dem Eigenvektor entspricht, welcher mit dem niedrigsten Eigenwert assoziiert ist.

7. Verfahren nach Anspruch 6, wobei das Molekül ein H2, LiH und/oder H2O Molekül ist.

8. Verfahren nach einem der Ansprüche 6 und 7, wobei die Anzahl an Qubits in der vorbestimmten Quantenschaltung einer zum Beschreiben des Moleküls verwendeten Anzahl an Spin-Orbitalen entspricht.

9. Verfahren nach einem der Ansprüche 1 bis 8, wobei das vordefinierte Stoppkriterium eine maximale Anzahl an Iterationen und/oder eine minimale Veränderung einer Varianz zwischen den Minima der mit dem Hamilton-Operator assoziierten Energie ist, welche nach zwei aufeinanderfolgenden Iterationen erhalten wird.

10. Verfahren nach einem der Ansprüche 1 bis 9, wobei die parametrischen Quantengatter Rotation-Quantengatter umfassen, und wobei die Parameter, welcher mit den Rotation-Quantengattern assoziiert sind, Winkelwerte umfassen.

11. Verfahren zum Bestimmen eines Quantenzustands eines chemischen Stoffes, beispielsweise eines Moleküls, umfassend ein Verfahren zum Auffinden eines optimalen Quantenzustands, welcher die mit einem Hamilton-Operator assoziierte Energie minimiert, mit einem Quantenprozessor und einem klassischen Prozessor nach einem der Ansprüche 1 bis 10, wobei ein Erwartungswert des Hamilton-Operators über einem gegebenen Quantenzustand der Energie des Quantenzustands entspricht.

12. Verfahren zum Bestimmen physikalischer Eigenschaften von Materialien, umfassend ein Verfahren zum Auffinden eines optimalen Quantenzustands durch Minimieren der mit einem Hamilton-Operator assoziierten Energie, mit einem Quantenprozessor und einem klassischen Prozessor nach einem der Ansprüche 1 bis 10.

**Revendications**

1. Procédé pour trouver un état quantique optimal minimisant l'énergie associée à un opérateur hamiltonien avec un processeur quantique et un processeur classique en utilisant une méthode de solveur propre quantique variationnel, dite méthode VQE, dans lequel l'opérateur hamiltonien représente une énergie d'une molécule, le processeur quantique comprenant un circuit quantique prédéterminé pour produire (S1) des états quantiques d'essai pour l'opérateur hamiltonien, ledit circuit quantique prédéterminé comprenant au moins des portes quantiques paramétriques associées à un ou plusieurs paramètres à optimiser, le procédé comprenant :

   - fournir (S2) l'opérateur hamiltonien sur une base orbitale et de manière itérative, jusqu'à ce qu'un critère d'arrêt prédéfini soit satisfait (S10) :
   - réaliser (S3, S4, S5, S6), par le processeur quantique conjointement avec le processeur classique, la méthode VQE pour trouver des valeurs optimisées pour au moins certains des paramètres associés aux portes quantiques paramétriques du circuit quantique prédéterminé qui donnent un état quantique optimal intermédiaire qui minimise l'énergie associée à l'opérateur hamiltonien,
   - mesurer (S7), sur le processeur quantique, une matrice densité réduite à une particule sur la base de l'état quantique optimal intermédiaire,
   - diagonaliser (S8), par le processeur classique, la matrice densité réduite à une particule pour obtenir une matrice de transformation, et déterminer une base orbitale mise à jour dans laquelle la matrice densité réduite à une particule est diagonale, sur la base de la matrice de transformation,
   - modifier (S9) l'opérateur hamiltonien en utilisant la matrice de transformation, pour exprimer l'opérateur hamiltonien dans la base orbitale mise à jour,

   dans lequel, lorsque le critère d'arrêt prédéfini est satisfait (S10), le procédé comprend en outre retourner (S11), en tant qu'état quantique optimal minimisant l'énergie associée à l'opérateur hamiltonien, à l'état quantique optimal intermédiaire qui minimise le plus l'énergie associée à l'opérateur hamiltonien.

2. Procédé selon la revendication 1, dans lequel l'étape de réalisation (S3, S4, S5, S6) la méthode VQE est un schéma itératif dans lequel le processeur quantique est utilisé conjointement avec le processeur classique, le processeur quantique préparant (S3) un état quantique d'essai pour l'opérateur hamiltonien et réalisant des mesures représentatives de l'énergie associée à l'opérateur hamiltonien pour ledit état quantique d'essai, et le processeur classique mettant à jour (S4) des valeurs des paramètres des portes quantiques paramétriques du circuit quantique prédéterminé sur la base des mesures réalisées par le processeur quantique, le schéma itératif étant exécuté jusqu'à ce qu'un deuxième critère d'arrêt prédéfini soit satisfait (S5), la méthode VQE retournant (S6) les valeurs optimisées des paramètres.

3. Procédé selon la revendication 1 ou 2, dans lequel le circuit quantique prédéterminé est un circuit quantique de produit comprenant uniquement des portes quantiques à un qubit sous la forme de rotations, un circuit quantique comprenant des portes quantiques de type fSim ou un circuit quantique de type approche de circuit à faible profondeur, dit LDCA.

4. Procédé selon l'une quelconque des revendications 1 à 3, dans lequel le procédé est appliqué sur un modèle de Hubbard pour lequel l'opérateur hamiltonien est fourni.

5. Procédé selon la revendication 4, dans lequel l'opérateur hamiltonien est un hamiltonien en seconde quantification.

6. Procédé selon l'une quelconque des revendications 4 à 5, dans lequel l'état quantique optimal correspond au vecteur propre associé à la plus faible valeur propre.

7. Procédé selon la revendication 6, dans lequel la molécule est une molécule H2, LiH et/ou H20.

8. Procédé selon l'une quelconque des revendications 6 et 7, dans lequel le nombre de qubits dans le circuit quantique prédéterminé correspond à un nombre d'orbitales de spin utilisé pour décrire la molécule.

9. Procédé selon l'une quelconque des revendications 1 à 8, dans lequel le critère d'arrêt prédéfini est un nombre maximal d'itérations et/ou un changement minimal d'une variance entre les minimums de l'énergie associée à l'opérateur hamiltonien obtenus après deux itérations consécutives.

10. Procédé selon l'une quelconque des revendications 1 à 9, dans lequel les portes quantiques paramétriques comprennent des portes quantiques de rotation, et dans lequel les paramètres associés auxdites portes quantiques de rotation comprennent des valeurs d'angles.

11. Procédé pour déterminer un état quantique d'un composé chimique, tel qu'une molécule, comprenant un procédé pour trouver un état quantique optimal minimisant l'énergie associée à un opérateur hamiltonien avec un processeur quantique et un processeur classique selon l'une quelconque des revendications 1 à 10, dans lequel une valeur d'attente de l'opérateur hamiltonien sur un état quantique donné correspond à l'énergie dudit état quantique.

12. Procédé pour déterminer des propriétés physiques de matériaux comprenant un procédé pour trouver un état quantique optimal en minimisant l'énergie associée à un opérateur hamiltonien avec un processeur quantique et un processeur classique selon l'une quelconque des revendications 1 à 10.

Diagonalization

QPU CPU

$$D^{(k)}_{ij} = \langle \psi_{\theta^{*(k)}} | C^{\dagger(k)}_i C^{(k)}_j | \psi_{\theta^{*(k)}} \rangle$$

$V^{(k)}$

$\theta^{*(k)}$

$H^{(k)}$

VQE

QPU

CPU

$\theta$

$$E_\theta = \langle \psi_\theta | H^{(k)} | \psi_\theta \rangle$$

# FIG. 1

| Providing an ansatz quantum circuit $U_\theta$, | S1 |

| Providing a Hamiltonian operator in an orbital basis ; Providing an encoding scheme Initializing parameters of the Hamiltonian and of the predetermined quantum circuit | S2 |

VQE

| Measuring the energy of the trial state associated with the Hamiltonian | S3 |

| Providing updated parameters of the circuit to minimize the energy | S4 |

S5

NO — Is the 2nd stopping criterion satisfied?

YES

| Returning parameters that minimize the energy associated with the Hamiltonian | S6 |

NATURAL ORBITALIZATION

| Measuring 1-RDM matrix | S7 |

| Diagonalizing the 1-RDM matrix, obtaining the associated transformation matrix, obtaining an updated orbital basis | S8 |

| Modifying parameters of the Hamiltonian function using the transformation matrix | S9 |

S10

NO — Is the 1st stopping criterion satisfied?

YES

| Returning the minimum energy of the Hamiltonian function | S11 |

FIG. 2

$q_0$ —— RY [$\theta_0$] ——

$q_1$ —— RY [$\theta_1$] ——

$q_2$ —— RY [$\theta_2$] ——

$q_3$ —— RY [$\theta_3$] ——

# FIG. 3A

$q_0$ ——————————————— $f$ Sim [$\theta_2$, $\theta_3$] ———

$q_1$ ———————— $f$ Sim [$\theta_0$, $\theta_1$] ————

$q_2$ —— X ——

$q_3$ —— X ——————————— $f$ Sim [$\theta_4$, $\theta_5$] ———

# FIG. 3B

FIG. 3C

FIG. 4A

FIG. 4B

FIG. 5A

FIG. 5B

FIG. 6A

FIG. 6B

FIG. 7A

FIG. 7B

**EP 4 102 416 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TKACHENKO et al.** Correlation-Informed Permutation of Qubits for Reducing Ansatz Depth in VQE. *arXiv:2009.04996*, 2020
- Quantum Theory of Many-Particle Systems. I. Physical Interpretations by Means of Density Matrices, Natural Spin-Orbitals, and Convergence Problems in the Method of Configurational Interaction. *Physical Review*, 15 March 1955, vol. 97 (6)

- Correlation-Informed Permutation of Qubits for Reducing Ansatz Depth in VQE. **NIKOLAY V TKACHENKO et al.** ARXIV.ORG. CORNELL UNIVERSITY LIBRARY, 10 September 2020
- **PIERRE-LUC DALLAIRE-DEMERS** ; **JONATHAN ROMERO** ; **LIBOR VEIS** ; **SUKIN SIM** ; **ALÁN ASPURU-GUZIK**. *Low-depth circuit ansatz for preparing correlated fermionic states on a quantum computer*, 04 January 2018